(19) Europäisches Patentamt
European Patent Office
Office européen des brevets

(11) **EP 0 905 229 B1**

(12) **EUROPÄISCHE PATENTSCHRIFT**

(45) Veröffentlichungstag und Bekanntmachung des Hinweises auf die Patenterteilung:
**28.04.2004 Patentblatt 2004/18**

(51) Int Cl.$^7$: **C12M 1/34**

(21) Anmeldenummer: **98116298.5**

(22) Anmeldetag: **28.08.1998**

(54) **Verfahren und Vorrichtung zur Ermittlung und Überwachung des physiologischen Zustandes mikrobieller Kulturen**

Process and device to determine and control the physiologic condition of microbial cultures

Procédé et dispositif pour déterminer et surveiller l'état physiologique de cultures microbiennes

(84) Benannte Vertragsstaaten:
**CH DE FR GB LI NL**

(30) Priorität: **01.09.1997 DE 19738078**

(43) Veröffentlichungstag der Anmeldung:
**31.03.1999 Patentblatt 1999/13**

(73) Patentinhaber: **Büchs, Jochen, Prof. Dr.-Ing.**
**52074 Aachen (DE)**

(72) Erfinder:
• **Büchs, Jochen, Prof. Dr.-Ing.**
**52074 Aachen (DE)**

• **Anderlei, Tibor**
**52064 Aachen (DE)**

(74) Vertreter: **Kohlmann, Kai, Dipl.-Ing.**
**Donatusstrasse 1**
**52078 Aachen (DE)**

(56) Entgegenhaltungen:
**WO-A-90/14414        WO-A-93/15402**
**WO-A-97/49985        DE-A- 2 926 138**
**DE-A- 4 445 668        DE-U- 9 003 081**
**US-A- 4 550 590**

## Beschreibung

Die Erfindung betrifft ein Verfahren zur Ermittlung und Überwachung des physiologischen Zustandes mikrobieller Kulturen durch quasikontinuierliche Messung der Atmungsaktivitäten in einem geschüttelten Meßkolben unter sterilen Bedingungen, wobei der Meßkolben in einer Spülphase zur Versorgung der mikrobiellen Kulturen mit Gas durchströmt wird, anschließend in einer Meßphase der Gasstrom in den Gasraum des Meßkolbens mit mindestens einem Absperrmittel unterbrochen und mit mindestens einem Meßwertgeber mindestens eine für die Atmungsaktivität aussagekräftige Meßgröße erfaßt wird, die nach Umsetzung in ein elektrisches Signal in einem Steuerrechner verarbeitet wird. Außerdem betrifft die Erfindung eine Vorrichtung zur Durchführung dieses Verfahrens.

Aus der DE 44 15 444 A1 ist ein derartiges Verfahren sowie eine Vorrichtung zu dessen Durchführung bekannt. Der Abfall des Sauerstoffpartialdruckes in dem Gasraum des über ansteuerbare Ventile geschlossenen Meßkolbens wird mittels einer sterilisierbaren $pO_2$-Elektrode gemessen und in dem Steuerrechner in die Sauerstofftransferrate (OTR) umgerechnet. Weitere für die Atmungsaktivität der Kultur aussagekräftige Meßgrößen lassen sich mit dem bekannten Verfahren und der Vorrichtung nicht ermitteln. Daher lassen sich einige, für die Ermittlung und Überwachung des physiologischen Zustandes der mikrobiellen Kultur wichtige Fragen, wie beispielsweise das Vorliegen einer Sauerstofflimitierung bzw. die Art der verstoffwechselten Kohlenstoffquelle, nicht beantworten.

Aus der DE-U-9003081 ist eine Vorrichtung zur Messung des Drucks in geschlossenen Reaktionsgefäßen bekannt, mit der "on-line" das Wachstum von Organismen im Reaktionsgefäß festgestellt werden kann. Der Drucksensor weist eine Trennmembran auf.

Aus der DE 44 45 668 A1 ist eine Sonde zur Messung des Partialdrucks von in Flüssigkeiten gelösten Gasen bekannt. An der Spitze des Kopfes der Sonde befindet sich ein Messraum mit einer Membran, die den Probenraum vom Messraum trennt. Im Probenraum befindet sich die Flüssigkeit mit dem zu bestimmenden Gas.

Aus der US-A-4,550,590 ist ein Apparat zur Messung der Gaskonzentration in einer Flüssigkeit bekannt. Der Apparat umfasst eine Druckmesseinrichtung, die in einem Gehäuse angeordnet ist, dass eine semi-permeable Membran aufweist. Das Gehäuse passt in ein Rohr, durch das die Flüssigkeit fließt, deren Gaskonzentration überwacht werden soll.

Nachteilig bei dem Verfahren nach der DE 44 15 444 A1 und der Vorrichtung zu dessen Durchführung ist, dass ein Ausfall der $pO_2$-Elektrode zum vollständigen Abbruch des Versuchs zwingt, da sie nicht gewechselt werden kann, ohne die Kultur zu kontaminieren. Zudem erfordert das bekannte Verfahren den Einsatz sterilisierbarer Meßwertgeber.

Außerdem ist die Genauigkeit der Messung vielfach nicht ausreichend, da zur exakten Berechnung der Sauerstofftransferrate (OTR) der Respirationsqoutient (RQ) möglichst während der gesamten Messung bekannt sein sollte.

Ausgehend von diesem Stand der Technik liegt der Erfindung die Aufgabe zugrunde, ein Verfahren und eine Vorrichtung zu dessen Durchführung vorzuschlagen, die die vorgenannten Nachteile nicht aufweist.

Die Aufgabe wird erfindungsgemäß durch die Merkmale des Anspruchs 1 und 8 gelöst.

Die erfindungsgemäße Maßnahme erhöht auf verblüffend einfache Art und Weise wirkungsvoll die Meßgenauigkeit des Verfahrens und der Vorrichtung. Dies ist besonders bei schwach atmenden mikrobiellen Kulturen wichtig.

Das Verfahren erlaubt erstmals den Einsatz nicht sterilisierbarer Meßwertgeber (Sterilisierbedingungen: T=121°C, p(absolut)=2bar), so daß nun auch kompaktere und damit leichtere Meßsonden verwendet werden können, die zudem wesentlich genauer arbeiten und eine bessere Auflösung aufweisen.

Ein weiterer Vorteil besteht darin, defekte Meßwertgeber während eines Versuchs auswechseln zu können, ohne die im Meßkolben befindliche mikrobielle Kultur zu kontaminieren.

Schließlich ist es aufgrund der steriltechnischen Trennung nicht mehr erforderlich, die Meßwertgeber während des Sterilisierens des Meßkolbens zwischen zwei Versuchen von ihrer Energieversorgung zu trennen. Infolgedessen entfällt die bei dem bisherigen Verfahren und der zugehörigen Vorrichtung notwendige Polarisationszeit der Meßwertgeber, so daß die Messung sofort nach Abschluß der Sterilisation gestartet werden können.

In einer Ausgestaltung der Erfindung nach Anspruch 2 wird neben der mit dem bekannten Verfahren bereits ermittelten Sauerstofftransferrate (OTR) nun auch gleichzeitig eine weitere für die Ermittlung und Überwachung des physiologischen Zustandes mikrobieller Kulturen wichtige Meßgröße, die Kohlendioxidtransferrate (CTR), erfaßt. Hierzu wird ein Kohlendioxidmeßwertgeber eingesetzt, der vorzugsweise nach dem Infrarotprinzip arbeitet.

Durch die erfindungsgemäße Maßnahme, den Gasraum steriltechnisch von dem Meßwertgeber zu trennen, ist alternativ der Gebrauch von hochpräzisen, jedoch nicht sterilisierbaren Miniaturdrucksensoren möglich. Mit dem Drucksensor bestimmt man den Gesamtdruck im Gasraum des Meßkolbens. Während der Meßphase kann sich der Gesamtdruck ändern, wenn z. B. die mikrobielle Kultur mehr Sauerstoff einatmet als Kohlendioxid ausatmet. Mit dem parallel gemessenen Sauerstoffkonzentrationsabfall oder Sauerstoffpartialdruckabfall als Meßgröße für die Sauerstofftransferrate (OTR) wird die Kohlendioxidkonzentrationsänderung, und damit die Kohlendioxidtransferrate (CTR) vom Steuerrechner ermittelt.

Die gleichzeitige Ermittlung der Kohlendioxidtransferrate (CTR) ermöglicht es, daß im Steuerrechner aus der gemessenen Sauerstofftransferrate sowie der Kohlendioxidtransferrate der Respirationsquotient der mikrobiellen Kultur errechnet wird. Aufwendige Fermentationen in gerührten Bioreaktoren (Fermentervolumen >1L, mit Abgasanalytik), die bisher für die Bestimmung des Respirationsquotienten (RQ) nötig waren, sind nun nicht mehr notwendig. Da geschüttelte Meßkolben einfacher zu handhaben sind(schnell sterilisierbar, kleine Füllmenge, kleines Gewicht in großer Zahl parallel betreibbar) als gerührte Fermenter, lassen sich bei der RQ-Bestimmung mit dem erfindungsgemäßen Verfahren Zeit und Kosten einsparen. Weiterhin liefert die laufende Berechnung des Respirationsquotients (RQ) fortlaufend Informationen, beispielsweise über die Art der Nährstoffquelle, die von den mikrobiellen Kulturen assimiliert wird bzw. über die gerade dominierende Stoffwechselreaktion.

Mit dem kontinuierlich errechneten Respirationsquotienten (RQ) kann außerdem die Zusammensetzung des Spülgases besser als bei dem bekannten Verfahren der Zusammensetzung in einem normalen, konventionellen Schüttelkolben angeglichen werden. Folglich lassen sich die Meßergebnisse des erfindungsgemäßen Verfahrens korrekt auf die Verfahren mit normalen konventionellen Schüttelkolben übertragen.

Schließlich bietet das erfindungsgemäße Verfahren in Ausgestaltung der Ansprüche 2 und 3 die Möglichkeit, nicht nur die Sauerstoff- und Kohlendioxidtransferrate zu ermitteln und den Respirationskoeffizienten zu errechnen, sondern auch eine Fermentation in geschüttelten Meßkolben nach einer dieser Größen zu regeln. Als Stellgröße kann beispielsweise die Gaszusammensetzung im Gasinnenraum fungieren.

Eine mikrobielle Kultur gerät in Sauerstofflimitierung, wenn die gelöste Sauerstoffkonzentration in der mikrobiellen Kulturlösung unter einen für diese Kultur kritischen Wert fällt. Die Sauerstofflimitierung wirkt sich wie folgt auf die mikrobielle Kultur aus:

- Die Kultur wächst langsamer, wird aber ansonsten nicht beeinflußt.
- Die Kultur schaltet teilweise auf einen anaeroben Stoffwechselweg um und bildet ungewollte Nebenprodukte (z.B. Lactat, EtOH, usw.).
- Die Kultur schaltet ihren Wertproduktstoffwechsel vollkommen ab oder um.
- Die Kultur stirbt ab.

Alle vier Fälle sind in der industriellen Fermentation nicht erwünscht. Daher ist es wichtig, eine Sauerstofflimitierung zu erkennen und zu vermeiden.

Die zur Beurteilung mikrobieller Kulturen wichtige Frage, ob eine Sauerstofflimitierung vorliegt, läßt sich mit Hilfe des erfindungsgemäßen Verfahrens in Ausgestaltung des Anspruchs 4 beantworten. Hierzu wird vorzugsweise in der Meßphase der Sauerstoffpartialdruck mit einer p02 - Elektrode gemessen und aus dem zeitlichen Verlauf des Signals der p02 - Elektrode mit Hilfe des Steuerrechners eine Sauerstofflimitierung signalisiert: Bei einem linearen Abfall des Signals liegt keine Sauerstofflimitierung vor. Eine logarithmische Form der Abfallkurve deutet indes auf eine Sauerstofflimitierung hin.

Eine weitere Steigerung der Meßgenauigkeit läßt sich erzielen, wenn durch eine Betriebsweise der herkömmlichen, an sich bekannten Magnetventile gemäß den Merkmalen des Anspruchs 5 deren schädliche Wärmeentwicklung verringert wird.

Die Wärmeentwicklung läßt sich außerdem dadurch reduzieren, wenn sowohl der Einlaß als auch der Auslaß für das Spülgas als Absperrmittel herkömmliche 3/2 Wege-Magnetventile aufweisen, die während der Meß- und der Spülphase mit dem Spülgas durchströmt werden.

Um die Dichtigkeit der Meßkolben zu testen, wird in einer weiteren Ausgestaltung der Erfindung vorgeschlagen, daß vor der Ermittlung und Überwachung des physiologischen Zustandes einer mikrobiellen Kultur der Meßkolben mit einem Spülgas, beispielsweise Stickstoff, gespült wird und nach dem Schließen der Absperrmittel, beispielsweise der Magnetventile, von Aus- und Einlaß mit mindestens einem der Meßwertgeber, beispielsweise einen Sauerstoffsensor, in Verbindung mit dem Steuerrechner eine etwaige Änderung der Zusammensetzung des Spülgases signalisiert wird.

Bleibt die Zusammensetzung des Spülgases gleich, ist der Meßkolben dicht und die Messung kann gestartet werden. Ändert sich die Zusammensetzung, müssen die Dichtungen der Meßkolben überprüft werden. Mit dieser Methode läßt sich zuverlässig sicherstellen, daß keine Meßwerte aufgrund von Undichtigkeiten der Meßkolben verfälscht werden.

Eine vorteilhafte Vorrichtung zur Durchführung des Verfahrens ergibt sich aus den Merkmalen des Anspruchs 8.

Wenn das Trennmittel als Sterilfilter ausgebildet ist und der Meßwertgeber in eine an dem Meßkolben angeordnete Halterung einsetzbar ist, ist es möglich, den Meßkolben ohne die Meßwertgeber zu sterilisieren. Erst nach der Sterilisation werden die Meßwertgeber wieder eingesetzt und sind sofort einsatzbereit.

Eine optische Erfassung der Gaskonzentration ermöglicht eine Vorrichtung nach Anspruch 10.

Das mit dem Schütteltablar zu bewegende Gewicht und die Kosten der Vorrichtung lassen sich reduzieren, indem der Auslaß für das Spülgas und das dem Auslaß zugeordnete Absperrmittel als Diffusionsrohr mit einer Mindestlänge von 10 mm ausgebildet ist. Außerdem reduziert sich die Störanfälligkeit der Vorrichtung durch den Fortfall eines mechanischen Absperrmittels im Auslaß.

Das Diffusionsrohr ist beim Schließen des Absperrmittels am Einlaß mit einem Gas bekannter Zusammensetzung gefüllt. Während der Messung finden Druck- und Konzentrationsausgleichsvorgänge zwischen Gasinnenraum und Umgebung statt, die im Steuerrechner berücksichtigt werden, so daß auch ohne ein Ventil im Auslaß genaue Atmungsraten bestimmt werden können. Das Diffusionsrohr kann eine Füllung, beispielsweise Watte, enthalten, die zugleich als Sterilbarriere für den Auslaß dient.

Eine weitere Steigerung der Meßgenauigkeit läßt sich erzielen, indem anstelle der herkömmlichen Magnetventile an sich bekannte Niedrig-Energie-Ventile, zum Beispiel Impuls- oder Niederwattventile, an dem Ein- und/oder Auslaß des Meßkolbens vorgesehen werden.

Der Einsatz von Impulsventilen reduziert eine unerwünschte Wärmeentwicklung an den Ventilen, da nur zum Schalten Energie benötigt wird. Da der Schaltvorgang nur Sekundenbruchteile dauert, wird fast keine Wärmeleistung vom Impulsventil abgegeben.

Um die störanfälligen, weil einer dauernden Belastung durch die Schüttelbewegung ausgesetzten, Kabelverbindungen zu den Absperrmitteln zu vermeiden, hat es sich als vorteilhaft herausgestellt, die Energie zu deren Betätigung von der ruhenden Umgebung auf das Schütteltablar drahtlos zu übertragen. Zur drahtlosen Energieübertragung kommt beispielsweise ein System aus einer Solarzelle und einer Lichtquelle oder ein induktives Energieübertragungssystem in Frage.

Die Übertragungssicherheit der elektrischen Signale von den Meßwertgebern zu dem Steuerrechner läßt sich dadurch erhöhen, daß Verstärker der Meßwertgeber und Analog/Digital-Wandler für die analogen elektrischen Signale der Meßwertgeber auf dem Schütteltablar angeordnet sind. Diese Maßnahme bewirkt, daß nur digitale elektrische Signale das Schütteltablar verlassen, die bekanntlich wesentlich unempfindlicher als die analogen Signale sind.

Die Betriebssicherheit der erfindungsgemäßen Vorrichtung läßt sich wirksam steigern, indem die Übertragung der elektrischen Signale der Meßwertgeber drahtlos ist, beispielsweise mit einem Infrarot-Sender-Empfänger-System oder einer Funkstrecke. Außerdem wird die Handhabung der Meßkolben durch eine drahtlose Signalübertragung vereinfacht, wenn die elektrischen Signale bereits vom Meßwertgeber ausgehend übertragen werden. Ein Verkabeln der Meßwertgeber entfällt, beispielsweise wenn man die Meßkolben von dem Schütteltablar nimmt, um die Kultur anzuimpfen.

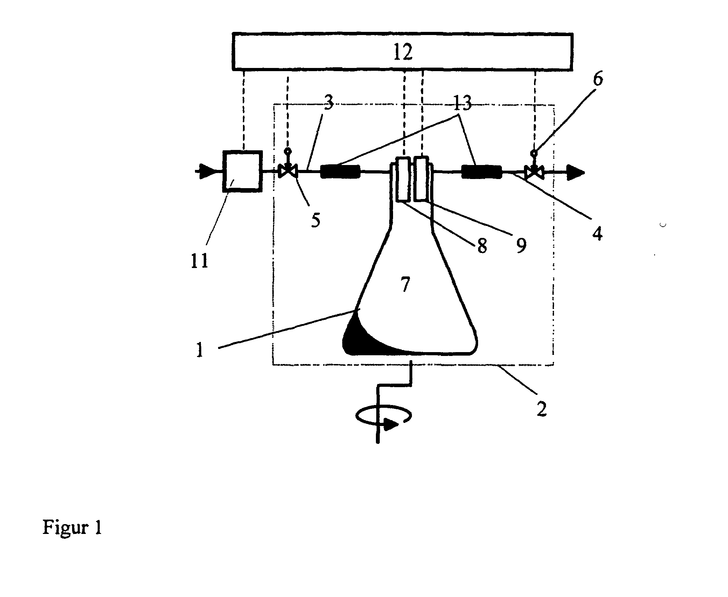

Nachfolgend wird die Erfindung anhand der Figuren 1 bis 5 näher erläutert:

Fig. 1 zeigt eine prinzipielle Darstellung einer Vorrichtung zur Durchführung des erfindungsgemäßen Verfahrens. Die Vorrichtung besteht im wesentlichen aus Meßkolben 1, die auf einem Schütteltablar 2 angeordnet sind. Jeder Meßkolben 1 besitzt einen Einlaß 3 sowie einen Auslaß 4 für ein Spülgas. Sowohl der Ein- als auch der Auslaß 3, 4 sind durch ein Magnetventil 5, 6 während der Meßphase verschließbar.

Oberhalb der Kultur sind an den Meßkolben 1 ein Meßwertgeber 8 in Form einer Sauerstoffmeßsonde sowie ein Meßwertgeber 9 in Form einer Kohlendioxidmeßsonde angeordnet. Zwischen beiden Meßwertgebern 8, 9 und dem Gasraum 7 ist ein in Figur 1 nicht erkennbares, jedoch detailliert in den Figuren 2 und 3 dargestelltes steriles Trennmittel angeordnet.

Die in Figur 1 gestrichelt dargestellten Linien deuten die Übertragungswege der elektrischen Signale der Meßwertgeber 8 bzw. 9 und einer Gasmischbatterie 11 sowie der Energie zu den Magnetventilen 5, 6 an. Die Verarbeitung der elektrischen Signale der Meßwertgeber 8, 9 sowie die Ansteuerung der Ventile 5, 6 und der Gasmischbatterie 11 erfolgt über einen Steuerrechner 12. Ferner befinden sich im Ein- und Auslaß 3, 4 Sterilfilter 13.

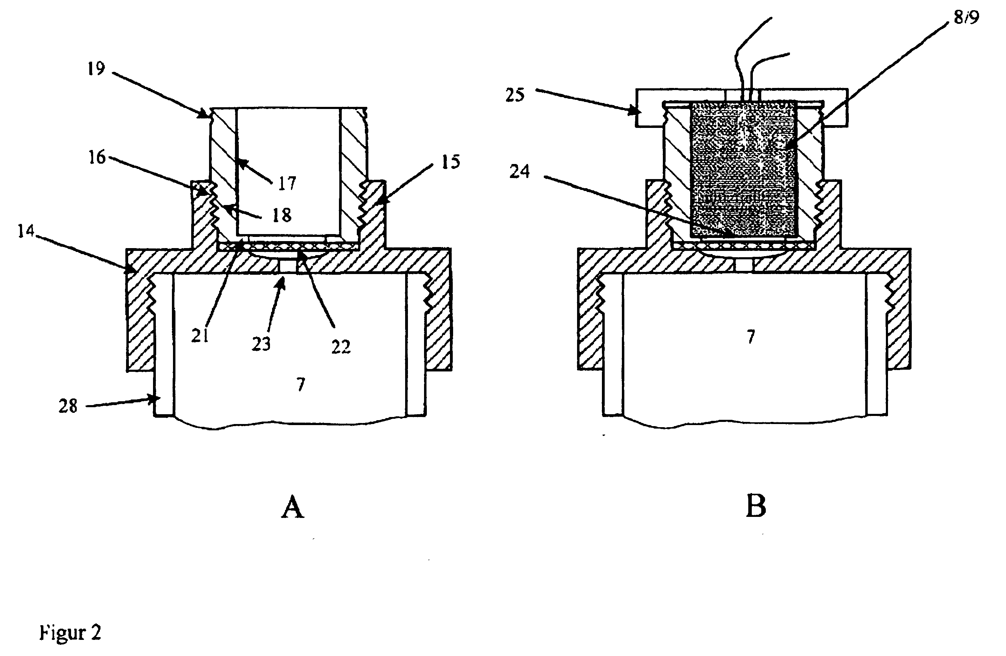

Die steriltechnische Trennung der Sauerstoffmeßsonde 8 wird nachfolgend anhand von Figur 2 erläutert. Außen auf einem Ansatz 28 des Meßkolbens 1 ist ein Verbindungsstück 14 aufgeschraubt. An dem Verbindungsstück 14 ist ein Stutzen 15 mit einem Innengewinde 16 angeformt, das ein zylindrisches Haltestück 17 mit jeweils an den Stirnseiten angeordneten Außengewinden 18, 19 aufnimmt. Vor dem Einschrauben des Haltestücks 17 in den Stutzen 15 wird ein handelsüblicher Sterilfilter 22 in den Stutzen 15 eingelegt. Wie insbesondere aus Figur 2 (A) ersichtlich, kommuniziert der Gasraum 7 über einen sich trichterförmig in Richtung des Sterilfilters 22 erweiternden Durchlaß 23 mit dem durch die Sauerstoffmeßsonde gasdicht abgeschlossenen Innenraum 24 des Haltestücks 17. Anschließend wird das Haltestück 17 stirnseitig mit einem Deckel 25 verschlossen.

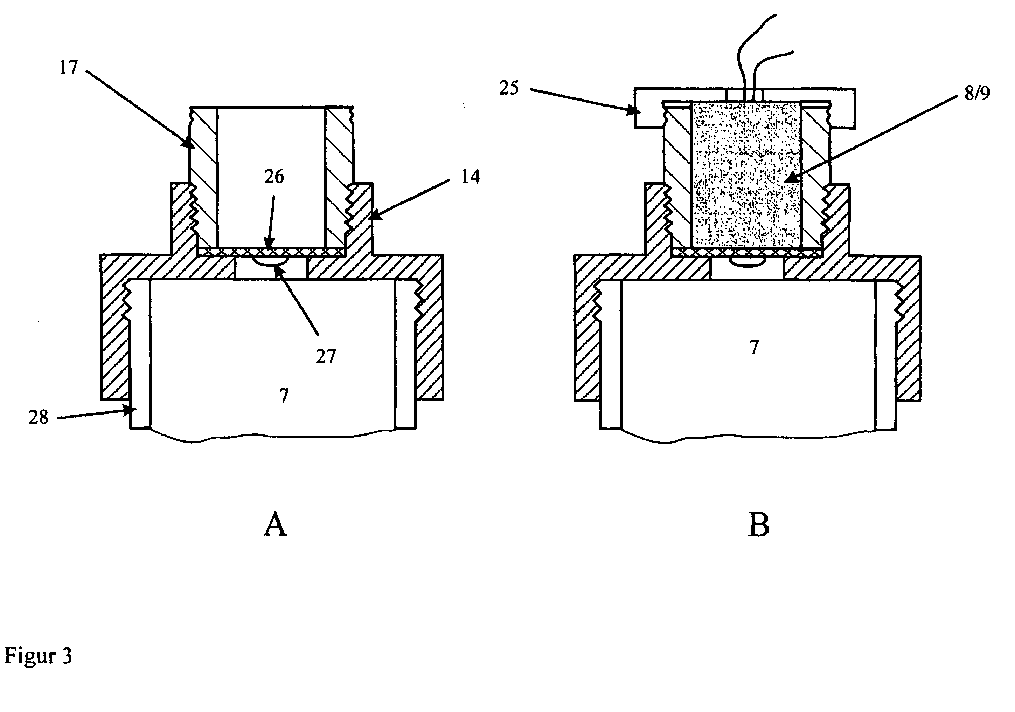

Die Ausführungsform nach Figur 3 unterscheidet sich von der nach Figur 2 darin, daß anstelle einer elektrochemischen Sauerstoffmeßsonde (8) ein optoelektronischer Meßwertgeber für die Bestimmung der Sauerstoffkonzentration Einsatz findet. Anstelle des Sterilfilters 22 ist das Trennmittel als eine Scheibe 26 ausgebildet auf deren dem Gasraum 7 zugewandten Seite eine Indikatorschicht 27 aus Fluoreszensfarbstoff dauerhaft aufgebracht ist. Ändert sich die Sauerstoffkonzentration im Gasinnenraum 7, reagiert die Indikatorschicht 27 mit einer Änderung der durch sie ausgesandten elektromagnetischen Strahlung im optischen Bereich. Damit ändert sich das von dem optoelekronischen Meßwertgeber 8 abgegebene elektrische Signal.

Wie bereits eingangs erwähnt, kann in einer Ausführung der Erfindung, wie sie Figur 1 zeigt, eine Sauerstofflimitierung erkannt werden, ohne die Gelöst-Sauerstoffkonzentration in der Kulturlösung direkt zu messen.

Die Sauerstofftransferrate (OTR) berechnet sich wie folgt:

$$OTR = \frac{dpo_2}{dt} \cdot \frac{Vg}{V_{fl} \cdot R \cdot T} \qquad \text{Formel 1}$$

| | | |
|---|---|---|
| dpO2/dt | Differentialquotient Sauerstoffpartialdruck über die Zeit | [bar/min] |
| $V_g$ | Gasvolumen des Meßkolbens | [ml] |
| $V_{fl}$ | Flüssigkeitsvolumen des Meßkolbens | [ml] |
| T | Temperatur | [K] |
| R | Gaskonstante | [bar*l/mol/K] |

Aus der Form der Abfallkurve des Sauerstoffpartialdrucks in der Meßphase läßt sich entnehmen, ob der Sauerstofftransport von der Sauerstoffverbrauchsgeschwindigkeit der Mikroorganismen (reaktionslimitiert) abhängt oder von dem Stoffübergang (Gasphase zu Flüssigphase) (stoffübergangslimitiert). Im ersten Fall ist der Sauerstoffverbrauch unabhängig von dem treibenden Partialdruckgefälle, d.h. der Differentialquotient aus der Formel 1 kann durch einen Differenzenquotient ersetzt werden:

$$\frac{\Delta po_2}{\Delta t} = OTR \cdot \frac{V_{fl} \cdot R \cdot T}{Vg} \qquad \text{Formel 2}$$

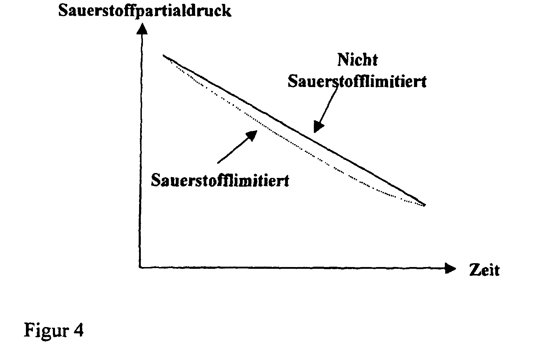

Formel 2 zeigt, daß bei einem linearen Sauerstoffpartialdruckabfall in der Meßphase keine Sauerstofflimitierung der Kultur vorliegt, wie dies das Diagramm in Figur 4 zeigt.

Liegt eine Sauerstofflimitierung vor (stoffübergangslimitiert), so ist der Sauerstoffverbrauch nicht mehr unabhängig von dem treibenden Partialdruckgefälle und die Gleichung für den Partialdruckabfall in der Meßphase sieht wie folgt aus:

$$\frac{ln\frac{po_{2_2}}{po_{2_1}}}{\Delta t} = k_L\alpha \cdot \frac{V_{fl} \cdot R \cdot T}{Vg \cdot He} \qquad \text{Formel 3}$$

| | | |
|---|---|---|
| $k_L$a | volumetrischer Stoffdurchgangskoeffizient | [1/h] |
| He | Henry'sche Konstante | [bar*l/mol] |
| $p_{O21}$ | Sauerstoffpartialdruck am Anfang der Meßphase | [bar] |
| $p_{O22}$ | Sauerstoffpartialdruck am Ende der Meßphase | [bar] |

Diese Abhängigkeit von dem treibenden Partialdruckgefälle führt zu einer nicht linearen Kurvenform (vgl. Figur 4).

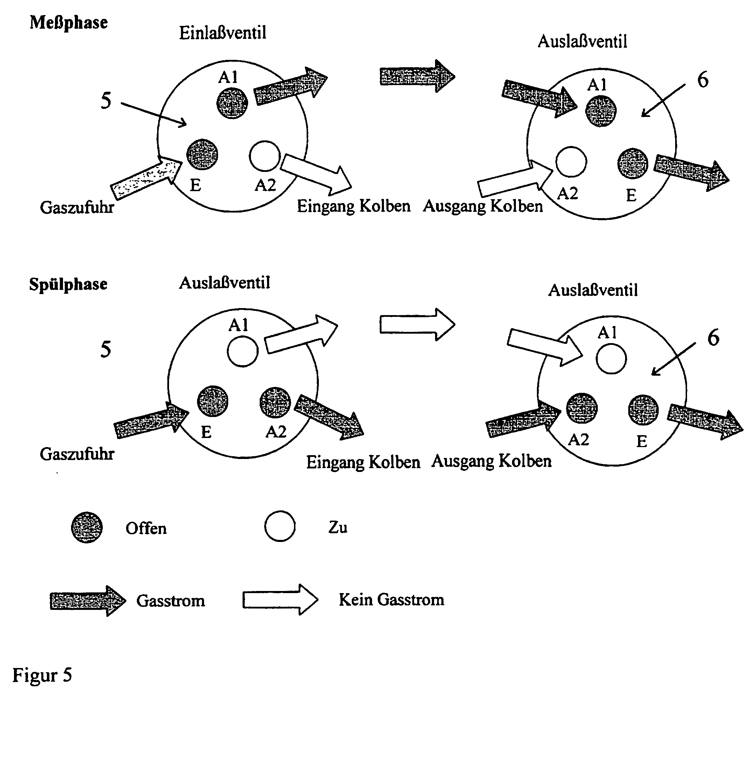

Werden an dem Ein- und Auslaß der Vorrichtung handelsübliche Magnetventile (3/2-Wegeventile) 5, 6, wie aus Figur 5 erkennbar, benutzt, kann durch Koppeln der Magnetventile 5, 6 eine Kühlwirkung erzielt werden. Hierzu verbindet man den Ausgang A1 (stromlos offen) des Magnetventils 5 am Einlaß 3 mit dem Ausgang A1 des Magnetventils 6 am Auslaß 4. Hierdurch wird sichergestellt, daß während der Meßphase (die Ventile sind stromlos) die Ventile 5,6 mit Gas durchströmt werden. Das Durchströmen der Ventile 5, 6 bewirkt, daß sie schneller abkühlen, da sie sich während der Spülphase (Ventile werden mit Strom versorgt) erwärmt haben. Durch dieses Verfahren, wird die Wärme nicht an den Gasraum 7 des Meßkolbens 1 abgegeben, sondern an das Spülgas, welches die Magnetventile 5, 6 durchströmt.

**Bezugszeichenliste:**

| | |
|---|---|
| Meßkolben | 1 |
| Schütteltablar | 2 |
| Einlaß Spülgas | 3 |
| Auslaß Spülgas | 4 |
| Magnetventil Einlaß | 5 |
| Magnetventil Auslaß | 6 |
| Gasraum | 7 |
| Meßwertgeber (Sauerstoffmeßsonde) | 8 |
| Meßwertgeber (Kohlendioxidmeßsonde) | 9 |
| -- | 10 |
| Gasmischbatterie | 11 |
| Steuerrechner | 12 |
| Sterilfilter (Einlaß / Auslaß) | 13 |
| Verbindungsstück | 14 |
| Stutzen | 15 |
| Innengewinde | 16 |
| Haltestück | 17 |
| Außengewinde | 18 |
| Außengewinde | 19 |
| -- | 20 |
| Stirnfläche | 21 |
| Sterilfilter | 22 |
| Durchlaß | 23 |
| Innenraum | 24 |
| Deckel | 25 |
| Scheibe | 26 |

| (fortgesetzt) | |
|---|---|
| Indikatorschicht | 27 |
| Ansatz am Glaskolben | 28 |

**Patentansprüche**

1. Verfahren zur Ermittlung und Überwachung des physiologischen Zustandes mikrobieller Kulturen durch quasikontinuierliche Messung der Atmungsaktivitäten in einem geschüttelten Messkolben (1) unter sterilen Bedingungen, wobei der Messkolben (1) in einer Spülphase zur Versorgung der mikrobiellen Kulturen mit Gas durchströmt wird, anschließend in einer Messphase der Gasstrom in den Gasraum (7) des Messkolbens mit mindestens einem Absperrmittel (5, 6) unterbrochen und mit mindestens einem Messwertgeber (8, 9) mindestens eine für die Atmungsaktivität aussagekräftige Messgröße erfasst wird, die nach Umsetzung in ein elektrisches Signal in einem Steuerrechner (12) verarbeitet wird, **dadurch gekennzeichnet, dass**

   - jede Messgröße durch eine steriles Trennmittel (22, 26) zwischen dem Gasraum (7) des Messkolbens (1) und dem Messwertgeber (8, 9) hindurch erfasst wird und
   - zum Auswechseln des Messwertgebers der Messwertgeber von dem Messkolben gelöst wird, während das Trennmittel (22, 26) am Messkolben verbleibt.

2. Verfahren nach Anspruch 1, **dadurch gekennzeichnet, dass** in der Messphase mit Hilfe eines ersten Messwertgebers (8) eine für die Sauerstofftransferrate (OTR) aussagekräftige Messgröße und mit Hilfe eines zweiten Messwertgebers (9) eine für die Kohlendioxidtransferrate (CTR) aussagekräftige Messgröße erfasst wird.

3. Verfahren nach Anspruch 2, **dadurch gekennzeichnet, dass** im Steuerrechner (7) aus der erfassten Sauerstofftrans-, ferrate sowie der Kohlendioxidtransferrate der Respirationsquotient (RQ) der mikrobiellen Kultur errechnet wird.

4. Verfahren nach einem der Ansprüche 1 bis 3, **dadurch gekennzeichnet, dass** in der Messphase der Sauerstoffpartialdruck mit dem ersten Messwertgeber erfasst und aus dem zeitlichen Verlauf des Signals des Messwertgebers mit Hilfe des Steuerrechners (7) eine Sauerstofflimitierung signalisiert wird.

5. Verfahren nach einem der Ansprüche 1 bis 4, **dadurch gekennzeichnet, dass** zumindest der Einlass (3) für das Spülgas in den Messkolben (1) als Absperrmittel ein Magnetventil (5) aufweist, bei dem nach dem Schaltvorgang zwischen Spül- und Messphase die hierzu erforderliche Schaltspannung auf eine Haltespannung reduziert wird.

6. Verfahren nach einem der Ansprüche 1 bis 5, **dadurch gekennzeichnet, dass** sowohl der Einlass (3) als auch der Auslass (4) für das Spülgas als Absperrmittel 3/2 Wege-Magnetventile aufweisen, die während der Mess- und der Spülphase mit dem Spülgas durchströmt werden.

7. Verfahren nach einem der Ansprüche 1 bis 6, **dadurch gekennzeichnet, dass** vor der Ermittlung und Überwachung des physiologischen Zustandes einer mikrobiellen Kultur der Messkolben (1) mit einem Spülgas gespült wird und nach dem Schließen der Absperrmittel (5, 6) von Aus- und Einlass (3, 4) mit mindestens einem der Messwertgeber (8, 9) in Verbindung mit dem Steuerrechner (7) eine etwaige Änderung der Zusammensetzung des Spülgases signalisiert wird.

8. Vorrichtung zur Durchführung des Verfahrens nach Anspruch 1 mit mindestens einem auf einem Schütteltablar (2) angeordneten Messkolben (1) mit einem Ein- und einem Auslass (3, 4) für ein Spülgas, wobei zumindest dem Einlass ein Absperrmittel (5) zugeordnet ist, mindestens einem Messwertgeber (8, 9) zur Erfassung einer aussagekräftigen Messgröße für die Atmungsaktivität einer mikrobiellen Kultur in dem Messkolben (1) sowie einem Steuerrechner (12) für die Verarbeitung der elektrischen Signale jedes Messwertgebers (8, 9), **dadurch gekennzeichnet, dass** zwischen jedem Messwertgeber (8, 9) und dem Gasraum (7) des Messkolbens (1) ein steriles Trennmittel (22, 26) an dem Messkolben (1) angeordnet ist und jeder Messwertgeber (8, 9) lösbar mit dem Messkolben (1) verbunden ist, während das Trennmittel am Meßkolben verbleibt.

9. Vorrichtung nach Anspruch 8, **dadurch gekennzeichnet, dass** das Trennmittel als Sterilfilter (22) ausgebildet ist und jeder Messwertgeber (8, 9) in eine an dem Messkolben (1) angeordnete Halterung (15, 17) einsetzbar ist.

10. Vorrichtung nach Anspruch 8 oder 9, **dadurch gekennzeichnet, dass** das Trennmittel als eine Scheibe (26) aus transparentem, jedoch gasundurchlässigem Material ausgebildet ist, auf deren dem Gasinnenraum (7) zugewandten Seite eine Indikatorschicht (27) dauerhaft aufgebracht ist, die auf Änderungen der Gaskonzentration im Gasinnenraum (7) mit einer Änderung der ausgesendeten elektromagnetischen Strahlung im optischen Bereich reagiert, und der Messwertgeber (8, 9) als optoelektronisches Bauelement ausgebildet ist.

11. Vorrichtung nach einem der Ansprüche 8 bis 10, **dadurch gekennzeichnet, dass** der Auslass (4) für das Spülgas als Diffusionsrohr mit einer Mindestlänge von 10 mm ausgebildet ist.

12. Vorrichtung nach einem der Ansprüche 8 bis 11, **dadurch gekennzeichnet, dass** zumindest im Einlass (3) für das Spülgas ein Niedrig-Energie-Ventil angeordnet ist.

13. Vorrichtung nach einem der Ansprüche 8 bis 12, **dadurch gekennzeichnet, dass** die Energieversorgung zur Betätigung der Absperrmittel (5, 6) drahtlos ist.

14. Vorrichtung nach einem der Ansprüche 8 bis 13, **dadurch gekennzeichnet, dass** die Übertragung der elektrischen Signale der Messwertgeber (8, 9) drahtlos ist.

## Claims

1. A process for determining and monitoring the physiological state of microbial cultures by quasi-continuous measurement of the respiratory activities in a shaken measuring flask (1) under sterile conditions, gas passing through the measuring flask (1) during a purge phase to supply the microbial cultures, the stream of gas being subsequently interrupted in a measuring phase in the gas chamber (7) of the measuring flask by means of at least one cut-off device (5, 6) and at least one measured variable meaningful regarding the respiratory activity being determined by means of at least one detecting element (8, 9), the measured variable being processed in a control computer (12) after conversion into an electrical signal,
   **characterised in that**

   - each measured variable is determined through a sterile separating device (22, 26) between the gas chamber (7) of the measuring flask (1) and the detecting element (8, 9) and
   - to replace the detecting element, the detecting element is detached from the measuring flask while the separating device (22, 26) remains on the measuring flask.

2. The process according to claim 1 **characterised in that**, during the measuring phase, a measured variable meaningful regarding the oxygen transfer rate (OTR) is determined by means of a first detecting element (8) and a measured variable meaningful for the carbon dioxide transfer rate (CTR) is determined by means of a second detecting element (9).

3. The process according to claim 2 **characterised in that** the respiration quotient (RQ) of the microbial culture is calculated in the control computer (7) from the oxygen transfer rate and the carbon dioxide transfer rate determined.

4. The process according to one of claims 1 to 3 **characterised in that**, in the measuring phase, the partial oxygen pressure is determined by means of the first detecting element and an oxygen limitation is signalled by means of the control computer (7) on the basis of the development in the signal of the detecting element over time.

5. The process according to one of claims 1 to 4 **characterised in that** at least the inlet (3) for the purge gas into the measuring flask (1) is equipped with a magnetic valve (5) as cut-off device in the case of which, following the control process between the purge and the measuring phase, the necessary control voltage is reduced to a retaining voltage.

6. The process according to one of claims 1 to 5 **characterised in that** both the inlet (3) and the outlet (4) for the purge gas are equipped with 3/2-way magnetic valves as cut-off devices, through which valves the purge gas flows during the measuring and purge phase.

7. The process according to one of claims 1 to 6 **characterised in that**, before determining and monitoring the physiological state of a microbial culture, the measuring flask (1) is flushed out with a purge gas and, following the closure of the cut-off devices (5, 6) of the outlet and the inlet (3, 4), any change in the composition of the purge gas is signalled by at least one of the detecting elements (8, 9) in association with the control computer (7).

8. A device for the execution of the process according to claim 1 with at least one measuring flask (1) arranged on a vibrator table (2), with an inlet and an outlet (3, 4) for a purge gas, a cut-off device (5) being allocated at least to the inlet, at least one detecting element (8, 9) for the detection of a measured variable meaningful for the respiratory activity of a microbial culture in the measuring flask (1) and a control computer (12) for processing the electrical signals of each detecting device (8, 9)
   **characterised in that**,
   between each detecting device (8) and the gas chamber (7) of the measuring flask (1), a sterile separating device (22, 26) is arranged on the measuring flask (1) and each detecting element (8, 9) is connected releasably to the measuring flask (1) while the separating device remains on the measuring flask.

9. The device according to claim 8 **characterised in that** the separating device has the form of a sterile filter (22) and each detecting element (8, 9) can be inserted into a retainer (15, 17) arranged on the measuring flask (1).

10. The device according to claim 8 or 9 **characterised in that** the separating device is formed as a disk (26) of a transparent yet gas-impermeable material on whose side facing the inner gas chamber (7) an indicator layer (27) is permanently applied which reacts to changes in the gas concentration in the inner gas chamber (7) by changing the electromagnetic radiation emitted in the optical range and the detecting element (8, 9) has the form of an optoelectronic structural element.

11. The device according to one of claims 8 to 10 **characterised in that** the outlet (4) for the purge gas is designed as a diffusion tube with a minimum length of 10 mm.

12. The device according to one of claims 8 to 11 **characterised in that** a low energy valve is arranged at least in the inlet (3) for the purge gas.

13. The device according to one of claims 8 to 12 **characterised in that** the energy supply for operating the cut-off device (5, 6) is wireless.

14. The device according to one of claims 8 to 13 **characterised in that** the transfer of the electrical signals from the detecting elements (8, 9) is wireless.

**Revendications**

1. Procédé pour déterminer et contrôler l'état physiologique de cultures microbiennes par une mesure quasi continuelle des activités respiratoires dans un ballon gradué agité (1) dans des conditions stériles, le ballon gradué (1) étant traversé par du gaz dans une phase de rinçage afin d'alimenter les cultures microbiennes, puis lors d'une phase de mesure, le flux gazeux étant interrompu dans la chambre d'expansion (7) du ballon gradué avec au moins un système de blocage (5, 6), et au moins une mesure éloquente pour l'activité respiratoire est relevée avec au moins un capteur de mesure (8, 9), laquelle mesure est exploitée dans un ordinateur de contrôle après avoir été convertie en un signal électrique, **caractérisé en ce que**

   - chaque mesure est relevée par un moyen de séparation stérile (22, 26) entre la chambre d'expansion (7) du ballon gradué (1) et le capteur de mesure (8, 9) et

   - pour changer le capteur de mesure, le capteur de mesure est enlevé du ballon gradué, alors que le moyen de séparation (22, 26) reste solidaire du ballon gradué.

2. Procédé selon la revendication 1, **caractérisé en ce que** dans la phase de mesure une mesure éloquente pour le taux de transfert d'oxygène (OTR) est obtenue au moyen d'un premier capteur de mesure (8) et **en ce qu'**une mesure éloquente pour le taux de transfert de dioxyde de carbone (CTR) est obtenue au moyen d'un second capteur de mesure (9).

3. Procédé selon la revendication 2, **caractérisé en ce que** le quotient respiratoire (QR) de la culture microbienne est déterminé dans l'ordinateur de contrôle (7) à partir du taux de transfert d'oxygène ainsi que du taux de transfert de dioxyde de carbone relevés.

4. Procédé selon l'une quelconque des revendications 1 à 3, **caractérisé en ce que** durant la phase de mesure, la pression partielle d'oxygène est relevée à l'aide du premier capteur de mesure et **en ce qu'**une valeur limite d'oxygène est signalée depuis l'évolution temporelle du signal du capteur de mesure au moyen de l'ordinateur de contrôle (7).

5. Procédé selon l'une quelconque des revendications 1 à 4, **caractérisé en ce qu'**au moins l'entrée (3) du gaz de rinçage dans le ballon de mesure (1) présente comme système de blocage une électrovanne (5), laquelle permet de réduire la tension de commutation requise à une tension de maintien après le procédé de commutation de la phase de rinçage et de la phase de mesure.

6. Procédé selon l'une quelconque des revendications 1 à 5, **caractérisé en ce qu'**aussi bien l'entrée (3) que la sortie (4) du gaz de rinçage présentent comme système de blocage des électrovannes à 3/2 voies, qui sont parcourus par le gaz de rinçage durant la phase de rinçage et de mesure.

7. Procédé selon l'une quelconque des revendications 1 à 6, **caractérisé en ce qu'**avant la détermination et le contrôle de l'état physiologique d'une culture microbienne le ballon gradué (1) est rincé à l'aide d'un gaz de rinçage et **en ce qu'**après la fermeture du système de blocage (5, 6) à l'entrée et à la sortie, toute modification de la composition du gaz de rinçage est signalée au moyen d'au moins un des capteurs de mesure en liaison avec l'ordinateur de contrôle.

8. Dispositif pour la réalisation du procédé selon la revendication 1 avec au moins un ballon gradué (1) agencé sur une table d'agitation (2) avec un entrée et une sortie (3, 4) pour un gaz de rinçage, un système de blocage (5) étant agencé au moins à l'entrée, au moins un capteur de mesure (8, 9) pour relever une mesure éloquente pour l'activité respiratoire d'une culture microbienne dans le ballon gradué (1) ainsi qu'un ordinateur de contrôle (12) pour le traitement des signaux électriques de chaque capteur de mesure (8, 9), **caractérisé en ce qu'**un moyen de séparation stérile (22, 26) est agencé au niveau du ballon gradué entre chaque capteur de mesure (8, 9) et la chambre d'expansion (7) du ballon gradué (1) et **en ce que** chaque capteur de mesure (8, 9) est raccordé de façon amovible avec le ballon gradué (1), alors que le moyen de séparation reste solidaire du ballon gradué.

9. Dispositif selon la revendication 8, **caractérisé en ce que** le moyen de séparation est formé comme un filtre stérile (22) et **en ce que** chaque capteur de mesure (8, 9) peut être placé dans un support (15, 17) agencé au niveau du ballon gradué (1).

10. Dispositif selon la revendication 8 ou 9, **caractérisé en ce que** le moyen de séparation est formé comme un disque (26) en un matériau transparent mais étanche au gaz, sur lequel une couche d'indicateur (27) est durablement appliquée, du côté tourné vers la chambre d'expansion (7) qui réagit aux changements de concentration de gaz par une modification du rayonnement électromagnétique renvoyé dans le domaine optique et **en ce que** le capteur de mesure (8, 9) est formé comme un élément optoélectronique.

11. Dispositif selon l'une quelconque des revendications 8 à 10, **caractérisé en ce que** la sortie (4) pour le gaz de rinçage est formée comme un tuyau de diffusion avec une longueur minimale de 10 mm.

12. Dispositif selon l'une quelconque des revendications 8 à 11, **caractérisé en ce qu'**au moins une vanne à basse énergie est agencée dans l'entrée (3) pour le gaz de rinçage.

13. Dispositif selon l'une quelconque des revendications 8 à 12, **caractérisé en ce que** l'alimentation énergétique pour activer le système de blocage (5, 6) est réalisée sans fil.

14. Dispositif selon l'une quelconque des revendications 8 à 13, **caractérisé en ce que** la transmission des signaux électriques des capteurs de mesure (8, 9) est réalisé sans fil.

Figur 1

Figur 2

A

B

Figur 3

**Sauerstoffpartialdruck**

**Nicht
Sauerstofflimitiert**

**Sauerstofflimitiert**

**Zeit**

Figur 4

**Meßphase**      Einlaßventil                     Auslaßventil

**Spülphase**      Auslaßventil                     Auslaßventil

Figur 5